# EUROPEAN PATENT SPECIFICATION

(11) **EP 1 242 099 B1**
(45) Date of publication and mention of the grant of the patent: **05.10.2011**
(21) Application number: 00988518.7
(22) Date of filing: 28.12.2000
(51) Int. Cl.: A61K 35/14, A61K 35/16, A61P 31/00, A61P 31/04, A61P 31/12, A61P 31/14, A61P 31/16, A61P 31/18, A61P 31/22, A61L 2/18, C12N 7/06

(54) **METHOD FOR INACTIVATING INFECTIOUS AGENTS IN BIOLOGICAL FLUIDS**
VERFAHREN ZUR INAKTIVIERUNG INFEKTIOESER ERREGER IN BIOLOGISCHEN FLÜSSIGKEITEN
PROCEDE RENDANT INACTIFS LES AGENTS INFECTIEUX DANS LES FLUIDES BIOLOGIQUES

(30) Priority: 23.12.1999 AU PQ486699
(43) Date of publication of application: 25.09.2002
(73) Proprietor: ELI LILLY AND COMPANY, Indianapolis, IN 46285 (US)
(72) Inventor: CHAM, Bill, Elliott, Sheldon, QLD 4157 (AU)
(74) Representative: Dey, Michael
(86) International application number: PCT/AU2000/001603
(87) International publication number: WO 2001/045718

(56) References cited:
- GB-A- 1 183 506
- US-A- 4 481 189
- US-A- 4 540 573
- US-A- 4 591 505
- US-A- 4 895 558
- US-A- 5 484 396
- FEINSTONE ET AL.: 'Inactivation of hepatitis B virus and non-A, non-B hepatitis by chloroform' INFECT. AND IMMUN. vol. 41, no. 2, 1983, pages 816 - 821, XP008038778
- HOROWITZ ET AL.: 'Viral safety of solvent/detergent-treated blood products' BLOOD COAGULATION AND FIBRINOLYSIS vol. 5, no. 3, 1994, pages S21 - S28, XP008038622
- CHAM ET AL.: 'A solvent systeem for delipidation of plasma or serum without protein precipitation' J. LIPID RESEARCH vol. 17, no. 2, 1976, pages 176 - 181, XP002904588
- CHAM ET AL.: 'Lipid apheresis: An in vivo application of plasma delipidation with organic solvents resulting in acute transient reduction of circulating plasma lipids in animals' J CLIN. APHERESIS vol. 10, no. 2, 1995, pages 61 - 69, XP008038621

## Description

The present invention relates to a method of inactivating an infectious agent, such as a micro-organism or virus, having a lipid envelope which may be present in a biological fluid including blood or blood products such as plasma. In particular, the present invention is directed towards a method of inactivating HIV, Hepatitis B or C virus.

The present invention will be described with particular reference to the HIV virus, however, it will be appreciated that the methods described herein may also be used for the treatment and Inactivation of other infectious agents having a lipid envelope or membrane and no limitation is intended thereby.

### BACKGROUND OF THE INVENTION

The disease known as Acquired Immune Deficiency Syndrome (AIDS) Is believed to be caused by a virus named Human Immunodeficiency Virus (HIV).

The HIV is an RNA virus. The free HIV virus or virion which circulates in the blood comprises a nucleoprotein core surrounded by a protective lipid envelope. In brief, the life cycle of the HIV virus begins with the HIV virus binding to the membrane of a target cell which is typically a human T4 lymphocyte or macrophage.

The lipid envelope has viral envelope glycoproteins which recognize and bind to CD4 receptors on a target cell surface. Following binding, the virus sheds its lipid envelope and penetrates the host cell. Reverse transcription generates a linear DNA copy of the viral RNA genome. The viral DNA is then integrated into the chromosomal DNA of the host cell. Expression of the integrated DNA generates viral mRNA that encodes regulatory and structural viral proteins. These viral proteins assemble at the host-cell surface. As they break through the host-cell membrane, the virus particles acquire a lipid envelope from its host which contains the envelope glycoprotein necessary for recognition and binding to an uninfected cell.

The amount of HIV circulating in the blood is known as the viral load. The viral load provides an indication as to how a patient is responding to the disease and assess the risk of progressing to AIDS. it is believed that the viral load has a direct relationship with the stages of the disease and reducing viral load has been shown to reduce the rate of disease progression. The current treatment regimes aim to reduce viral load by targeting the reproductive cycle of the cell borne virus. These therapies are ineffective against the mature virus circulating in the blood.

Antiviral drugs for use in the treatment of HIV have been designed to prevent or inhibit viral replication and typically target the initial attachment of the virus to the T-4 lymphocyte or macrophage, the transcription of viral RNA to viral DNA and the assemblage of the new virus during reproduction.

A major difficulty with existing HIV treatments is the high mutation rate of the virus. An individual may carry a number of different HIV strains, some of which may be resistant to some of the antiviral drugs. During treatment resistant strains may evolve. The difficulties associated with different mutations of the HIV virus has been attempted to be addressed by using a combination of drugs which must be taken according to strict protocols in order to be effective. This introduces difficulties with compatibility and compliance. Still further, many drugs have undesirable side effects.

Inactivation of viruses having a lipid envelope by treatment with chemical agents is known. The sensitivity of these virus to organic solvents has been used as a criteria for virus classification. Chloroform has been observed to be a particularly effective agent for inactivating lipid coated viruses. However, chloroform also denatures plasma proteins and is therefore quite unsuitable for use with fluids which are to be administered to an animal. Plasma proteins include coagulation factors II, VII, IX, X, plasmin, fibrinongen (Factor I), IgM, hemoglobin and interferon. Loss of these proteins will have adverse effects on a patient's health and may even lead to patient death. β-propiolactone is another solvent, which although inactivates lipid coated viruses also inactivates up to 75% of the blood protein factor VIII a critical protein for coagulation.

It should therefore be appreciated inactivation of viruses in biological fluids which are to be administered to an animal is quite distinct from simply sterilising fluids and surfaces. This is due to the presence of desirable proteinaceous components in biological fluids. An important use of plasma is in the treatment of patients with deficiencies of coagulation factors. Plasma with low levels of factor VIII is unsuitable for such therapeutic treatment. Further, administration of large amounts of denatured proteins to a patient may initiate an immune response which can in turn lead to autuimmune diseases or antibody to the denatured factor VIII itself.

Diethyl ether has also been proposed as a suitable solvent for inactivation of viruses having a lipid envelope. One reason for choosing diethyl ether is that from its early use as a general anaesthetic it is known to be generally nontoxic. However, diethyl ether is a relatively poor lipid solvent, especially for amphiphilic molecules such as phospholipids which form part of the viral lipid envelope. Some viruses such as poxviruses have been found to be potentially resistant to diethyl ether. Further, diethyl ether has a boiling point of 34°C which is less than the temperature of human blood. Therefore, contacting diethyl ether with a freshly withdrawn blood product will result in undesirable vaporisation of the ether.

In an effort to improve the liquid solubilizing properties of diethyl ether, it has been proposed to combine the diethyl ether with a non-ionic detergent. A detergent available under the tradename TWEEN 80 has been particularly preferred. The detergent increases the contact between the ether and lipids.

Di or trialkylphosphates have also been proposed as virus inactivating agents and have been observed to be superior to diethyl ether In this respect. A disadvantage of the phosphates is their low water solubility (less than 0.4%). Thus, In order for these agents to operate effectively, it is necessary to use non-ionic detergents such as the aforementioned TWEEN 80. However, the use of a detergent necessitates tedious procedures for removal thereof.

Procedures which have been proposed to remove non-ionic detergents include diafiltration using microporous membranes which retain plasma proteins, absorption of desired plasma components on chromatographic or affinity chromatographic supports and precipitation of plasma proteins.

The alkyl phosphate/detergent solvent system (SD) has achieved wide acceptance since its development in the mid 1980's and is used by the American Red Cross for treating plasma to inactivate HIV, Hepatitis B & C.

The indications for use of plasma treated by the SD method is limited and includes treatment for patients with deficiencies of coagulation factors for which there are no concentrate preparations available, acquired multiple coagulation factor deficiencies, reversal of warfarin effect and treatment of patients with thrombotic thrombocytopenic purpura.

In the SD process method, fresh frozen plasma (FFP) is thawed and filtered before polling and treatment with 1% tri (n-butyl) phosphate and 1 % Triton X-100 detergent for 4 hours at 30°C. The solvent/detergent system is removed by soybean oil extraction and reverse-phase chromatography on C18 resin. Water is removed by ultrafiltration and the plasma is finally sterile filtered.

It can be seen that solvent/detergent removal is a long and tedious batch process and in order to be able to operate effectively on a commercial scale it must be conducted on a large scale with relatively large volumes of pooled plasma. The SD method could not be used for continuous treating plasma from a single patient for ultimate return to the same patient. Further, the economics of treating small units of plasma on a single patient would be prohibitive.

As mentioned above, the major use of SD treated plasma is for treatment of patients having deficiencies in various coagulation factors which cannot be administered in other forms. Thus although it is critical that there is minimal denaturation of clotting factors, denaturation of other blood proteins may be tolerated. However, denaturation of these other blood proteins is not acceptable for large scale replacement of plasma.

As discussed above, reintroduction of plasma in which plasma proteins have been denatured can be toxic to a patient and in some cases, may even lead to patient death. For these reasons, treatment of an infected patient by viral inactivation of fluids such as plasma, although proposed, has not been adopted presumably in view of the health risks to a subject.

One proposal for reintroducing virus inactivated plasma to a patient has been described in US 5484396. In this method, blood is withdrawn from the patient and separated into a first component including red cells and platelets and a second infected component including plasma, white cells and cell free virus. The infected component is then exposed to diethyl ether for 5 or 10 minutes. Ether is removed by distillation at 50 - 52°C and the treated plasma with killed cell free virus and killed infected cells are returned to the patient

This patent also suggests the use of other solvents including chloroform. However, as mentioned above, chloroform is unacceptable as it denatures plasma proteins.

There are a number of difficulties associated with the method described in US 5484396. First, the method involves killing all the removed white cells and returning the killed cells to the patient. These killed cell fragments may be toxic. Any toxicity may be particularly dangerous for patients at an advanced stage of disease. Secondly, the ether is removed by distillation. This means that any lipids dissolved in the ether remain In the plasma. Plasma lipids are normally associated with proteins. Contact with organic solvents disrupts this association. Once disrupted, the lipids and proteins will not reassociate. Thus, in this method disassociated lipids are also returned to the patient. Again there are concerns regarding potential toxicity of disassociated plasma lipids.

Still further, ether is removed by distillation at about 50 - 52°C, although the patent does not describe the length of time the plasma is subjected to heating. The HIV virus is known to be heat sensitive. Thus it is unclear as to whether it is the ether, heating or a combination thereof which is responsible for the observed virus inactivation. Still further, it is generally recognised that the maximum temperature to which blood and plasma can be exposed is about 40°C. At higher temperatures denaturation of plasma proteins can occur.

It has been proposed to inactivate HIV in blood by heat treatment However, this method has not been adopted due to difficulties associated with adverse effects of high temperature on blood constituents.

As mentioned earlier, ether has already been proposed as an agent for inactivating lipid coated viruses in plasma. However, this solvent was not adopted as the rate of virus inactivation was shown to be superior with tri(n-butyl) phosphate (TNBP). US 4540573 provides some comparative date for viral inactivation by diethyl ether and TNBP. The viruses studied were Sinbis, Sendal and VSV viruses which are typical lipid containing viruses. These results show that treatment with diethyl ether at 4°C took many hours to inactivate these viruses. US 4481189 describes Inactivating Hepatitis B virus by contacting plasma with diethyl ether for 16 hours at 4°C. US 4540573 also includes studies of virus Inactivation by TNBP at room temperature. The minimum time for Inactivation of the viruses is 2 hours. It is also noted that the commercial plasma sterilisation procedure using TNBP Is carried out over 4 hours.

It is acknowledged that the earlier diethyl ether studies were conducted at 4°C whereas the studies of US 5484396 were conducted at room temperature. Nevertheless, although direct comparison between the diethyl ether experiments is not possible, the claim in US 5484396 that complete Inactivation of the HIV virus after 5 minute contact with diethyl-ether is remarkable. There are two possible conclusions which can be made from this observation. First, that the HIV virus is significantly more sensitive to diethyl ether than other viruses. In this case, the method as described in US 5484396 would not be suitable for treatment of patients infected with other lipid containing viruses such as Hepatitis B or C. This is unsatisfactory as it often happens that a patient is co-infected with Hepatitis C and HIV viruses and it would be desirable to be able to treat the patient for both conditions. Further, the method of US 5484396 would be quite unsuitable for sterilisation of blood products as it would be ineffective against non HIV viruses.

Alternatively, the diethyl ether may not be responsible for virus activation and the virus is inactivated during the distillation step when the plasma is heated to about 50 - 52°C. In this case, heating would appear to be an essential feature of the method. However, prolonged exposure of blood products to above 40°C can adversely affect blood components.

A further method for treating and introducing plasma to a patient is plasmapheresis (plasma exchange therapy) in which a patient's plasma is replaced with donor plasma or more usually a plasma protein fraction. This treatment can result in possible complications due to the possible Introduction of foreign proteins and transmissions of infectious diseases. This can be quite significant for patients with a comprised immune system such as patients with HIV. Still further plasmapheresis will also remove desirable elements in a patients' plasma including antibodies, and any anti-viral drugs circulating in the plasma.

US-A-5484396 discloses an apparatus and a method for the elimination of envelope viruses from plasma by contacting said plasma with a solvent comprising an ether at a concentration of 10% or more.

US-A-4895558 teaches a method for delipidating plasma and when the organic solvent containing the extracted lipids is removed from the aqueous phase by extraction with an organic solvent.

Cham et al. (Lipid research, vol. 17, no. 2, 1976, pages 176-181) describes a method for delipidating plasma or serum which involves the use of mixtures of the isopropyl ether and butanol/DIPE proportions of 25:75, 35:65 or 40:60.

US-A-4591505 discloses a solvent system for inactivating hepatitis B virus in mammalian blood plasma.

### SUMMARY OF THE INVENTION

The present invention is defined by the appended set of claims. Also described is a method of inactivating

Infectious agents having a lipid envelope or membrane which may at least partially overcome the above disadvantages.

According to a first broad form of the invention, there is provided a method of treating biological fluids containing an infectious agent having a lipid envelope or membrane, which may be exemplified by separating blood cells from plasma, contacting the plasma with a solvent system comprising a solvent in which lipids are soluble and in which hematological and biochemical constituents are substantially stable, for a time sufficient to reduce active levels of the infectious agent in the plasma, and separating the plasma the plasma from the solvent system whereby dissolved lipids are separated with and remain in the solvent system.

The treated plasma can be re-mixed with the blood cells although in some cases this may not be desirable or necessary.

Viral infectious agents which may be inactivated by the above system include lipid encoded viruses of the following genuses:
*Alphavirus* (alphaviruses), *Rubivuros* (rubella virus), *Flavivirus* (Flaviviruses), *Pestivirus* (mucosal disease viruses), (unnamed, hepatitis C virus), *Coronavirus*, (Coronaviruses), *Torovirus*, (toroviruses), *Arteivirus*, (arteriviruses), *Paramyxovirus*, (Paramyxoviruses), *Rubulavirus* (rubulavriuses), *Morbillivirus* (morbillirviruses), *Pneumovirinae* (the pneumoviruses), *Pneumovirus* (pneumoviruses), *vesiculovirus* (vesiculoviruses), *Lyssavirus* (lyssaviruses), *Ephemerovirus* (ephemeroviruses), *Cytorhabdovirus* (plant rhabdovirus group A), *Nucleorhabdovirus* (plant rhabdovirus group B), *Filovirus* (filoviruses), *influenzavirus A*, *B* (influenza A and B viruses), *influenza virus* C (influenza C virus), (unnamed, Thogoto-like viruses), *Bunyavirus* (bunyaviruses), *Phlebovirus* (phleboviruses), *Nairovirus* (nairoviruses), *Hantavirus* (hantaviruses), *Tospovirus* (tospoviruses), *Arenavirus* (arenaviruses), unnamed mammalian type B retroviruses, unnamed, mammalian and reptilian type C retroviruses, unnamed, type D retroviruses, *Lentivirus* (lentiviruses), *Spumavirus* (spumaviruses), *Orthohepadnavirus* (hepadnaviruses of mammals), *Avihepadnavirus* (hepadnaviruses of birds), *Simplexvirus* (simplexviruses), *Varicellovirus* (varicelloviruses), *Betaherpesvirinae* (the cytomegaloviruses), *Cytomegalovirus* (cytomegaloviruses), *Muromegalovirus* (murine cytomegaloviruses), *Roseolovirus* (human herpes virus 6), *Gammaherpesvirinae* (the lymphocyte-associated herpes viruses), *Lymphocryptovirus* (Epstein-Bar-like viruses), *Rhadinovirus* (saimiri-ateles-like herpes viruses), *Orthopoxvirus* (orthopoxviruses), *Parapoxvirus* (parapoxvinrses), *Avipoxvirus* (fowlpox viruses), *Capripoxvirus* (sheeppoxlike viruses), *Leporipoxvirus* (myxomaviruses), *Suipoxvirus* (swine-pox viruses), *Molluscipoxvirus* (molluscum contagiosum viruses), *Yatapoxvirus* (yabapox and tanapox viruses), Unnamed, African swine fever-like viruses, *iridovirus* (small iridescent insect viruses), *Ranavirus* (front iridoviruses), *Lymphocystivirus* (lymphocystis viruses of fish),

These viruses include the following human and animal pathogens:
Ross River virus, fever virus, dengue viruses, Murray Valley encephalitis virus, tick-borne encephalitis viruses (including European and far eastern tick-bome encephalitis viruses, hepatitis C virus, human coronaviruses 229-E and OC43 and others (causing the common cold, upper respiratory tract infection, probably pneumonia and possibly gastroenteritis), human parainfluenza viruses 1 and 3, mumps virus, human parainfluenza viruses 2, 4a and 4b, measles virus, human respiratory syncytial virus, rabies virus, Marburg virus, Ebola virus, influenza A viruses and influenza B viruses, *Arenaviruss:* lumphocytic choriomeningitis (LCM) virus; Lassa virus, human immunodeficiency viruses 1 an 2, hepatitis B virus, Subfamily: human herpes viruses 1 and 2, herpes virus B, Epstein-Barr virus), (smallpox) virus, cowpox virus, molluscum contagiosum virus.

The above method is particularly suited for reducing the viral load of HIV, Hepatitis B or C.

An advantage of the present method is that the present method is non-specific and may remove or inactivated any lipid coated virus, including drug resistant strains. The method of the present invention may find particular application for resistant HIV strains.

Suitable solvent systems comprise at least one alcohol, ether, amine, ester or mixture thereof. Preferable solvents are ethers or mixtures of alcohols with ethers. The alcohols suitably include those which are not appreciably miscible with plasma or other biological fluids and these can include lower alcohols including C4 to C8 alcohols. Preferred are the butanols (butan-1-ol)- and (butan-2-ol). C2-5 ethers are also preferred and these can include the propyl ethers (di-isopropyl ether (DIPE), ethyl propyl ether propyl ether), diethyl ether or a mixture thereof. Other solvents which may be applicable can include amines, esters, hydrocarbons such as hexane and mixtures. Especially preferred solvents are those which can (1) rapidly disrupt the viral lipid envelope or irreversibly denature the other viral constituents, (2) is substantially immiscible with plasma or other biological fluids, (3) can be quickly removed from plasma or other biological fluids (if required), and (4) does not denature hematological or biochemical constituents of plasma to an extent which may be toxic to an animal to which the plasma may be introduced. Preferred solvent systems include butanol, diisopropyl ether, diethyl ether or a mixture thereof and these may be in the ratio of 0% to about 60% of the alcohol with about 40% to about 100% of the ether. Preferably, the solvent system comprises between 0 to about 50% alcohol and between about 50 to about 100% ether.

The time during which the plasma is in contact with the solvent system is the same extent dependent upon the effectiveness of the solvent system. Typically the contact time is between about 5 seconds to about 2 hours, preferably between about 30 seconds to one hour and most preferably between about 5 or about 10 minutes to about 30 minutes.

Particularly suitable solvent systems for inactivating an infectious agent having a lipid envelope or membrane comprise between about 40 to about 100% di-isopropyl ether or di-ethyl ether and between about 0 to about 60% butanol.

According to a further broad form of the invention, there is provided a method of reducing the activity of an infectious agent having a lipid envelope or membrane in a fluid or blood product, the method comprising contacting the fluid or blood product with a solvent system comprising a solvent in which lipids are soluble and in which hematological and biochemical constituents are substantially stable and which is immiscible with the fluid or blood product, for a time to reduce active levels of the infectious agent, and separating the fluid from the solvent system whereby dissolved lipids are separated with and remain in the solvent system, characterized in that the solvent system comprises at least one alcohol, ether, hydrocarbon, amine, ester or mixture thereof.

It will be appreciated that fluids treated in this manner are not limited to plasma. The method may be used to reduce active viral levels in any fluid or composition carrying active infectious agents having a lipid envelope or membrane. Typical fluids include mammalian blood plasma, pooled plasma, avarian blood plasma, blood plasma fractions, blood cell derivatives such as haemoglobin, alphainterferon, T-cell growth Factor, platelet derived growth Factor and the like, plasminogen activator, blood plasma precipitates including cryoprecipitate, ethanol precipitate and polyethylene glycol precipitate, or supernatants such as cryosupematant, ethanol supematent and polyethylene glycol supernatent, mammalian semen and serum.

Preferred solvent systems are those described above. The method is accordingly suitable for reducing levels of active infectious agents in pooled blood, blood plasma and plasma fractions and can provide an alternative to the SD plasma treatment as described above.

Other uses include sterilisation of fluids used in tissue cultures such as foetal calf serum. Foetal calf serum (FCS) is used for culturing cells for research and vaccine production. FCS contaminated with cattle pestivirus when used for vaccine production for ruminant animals can give rise to actual disease in the field. At present the only way to address the problem is to maintain a pestivirus free herd or to individually test each foetus. Both approaches are very expensive. There is a need in the industry for an economical and effective method of producing pestivirus free FCS.

Suitable fluids for treatment by the above method also include products from cancer or normal cells or from fermentation processes following gene-insertion.

In both the above methods, the solvent system may be separated from the fluid being treated by any suitable manner, and it is preferred that the separation does not adversely affect any biochemical or hematological constituents of the fluid. As the solvents are substantially immiscible in the aqueous fluid, the separation is typically achieved by allowing the two layers to separate and removing the relevant layer, depending upon whether the solvent system is more or less dense than the aqueous phase. An advantage of separation in this manner is that dissolved lipids in the solvent layer can also be removed. In this way, lipid fragments which may be toxic can be substantially removed from the fluid. Separation of the two phases may be facilitated by centrifugation. Alternatively, at least part of the solvent may be removed by distillation, preferably under reduced pressure.

The fluid after separation may still comprise some entrained solvent which is usually in the form of an emulsion. The fluid may therefore be treated with a de-emulsifying agent The de-emulsifying agent may comprise ether or another agent and a preferred ether is di-ethyl ether. The ether may be added to the fluid, or alternatively the fluid is dispersed in the ether. The ether can be removed by similar methods as described above in relation to separation of the solvent.

In the method of the present invention, the plasma may be treated in a continuous or discontinuous basis. Typically, in a continuous method blood from an animal may be withdrawn mixed with an anti- coagulant solution and centrifuged to separate blood cells. The plasma is mixed with a solvent system according to the invention which may be a butanol-DIPE (40%-60%)V/V) or 100% DIPE solution. The plasma and solvent are mixed before being passed to a plasma solvent separation unit where most of the solvent (organic phase) is removed from the plasma (aqueous phase). The separation unit may be a simple unit having a lower outlet through which the denser aqueous phase may pass. Ether which breaks down any emulsion In the plasma is typically added to the plasma from the separation unit The plasma may then be pumped through a second centrifugal separator where the balance of the solvents, and ether, are removed. The treated plasma is drawn by a fluid replacement pump to be mixed with the blood cells, if required. (A replacement fluid may be added, as required, to the plasma to overcome any loss in bulk of the plasma during the treatment and separation steps.)

In a discontinuous method of treatment, plasma is typically treated at a site remote from the patient with the discontinuous method, multiple washings with ether can be conducted.

It will be appreciated that various modifications may be made to the treatment and separation steps as described above. For example, treatment of the plasma with the solvent may be facilitated by dispersing the solvent or plasma in the other of the plasma or solvent. Such dispersion may be accomplished by means of a spinning chamber. An example of a suitable arrangement is described in US 5744038.

### DETAILED DESCRIPTION OF AN EMBODIMENT EXPLAINING THE INVENTION

The Invention is not directed to the treatment of a patient.

### Experiment 1

The influence of treatment of plasma on the biochemical and haematological constituents was studied in animals.

Approximately one-fourth of the blood volumes of the animals were removed. The blood cells were removed from the plasma by centrifugation. The plasma was treated, then remixed with the blood cells and re-introduced back into the original animal by intravenous infusion. Blood samples were collected before and after this procedure for biochemical, haematological and lipid analyses.

During the experimental period there were no changes in the following biochemical and haematological parameters.

| **Biochemical** | **Haematological** |
|---|---|
| Bilirubin | WBC |
| Total protein | RBC |
| Albumin | Haemaglobin |
| Total globulin | Hct |
| alpha₁, alpha₂, beta | MCV |
| and gamma blobulins | MCH |
| Sodium | MCHC |
| Potassium | Polymorphs |
| Chloride | Lymphocytes |
| Total Carbon dioxide | Monocytes |
| Calcium | Eosinophils |
| Phosphate | Platelets |
| Urea | |
| Urate | |
| Creatinine | |
| Alkaline Phosphatase | |
| Lactate dehydrogenase | |
| Aspartate transaaminase | |
| Creatine hinase | |
| Amylase | |
| 5'Nucleotidase | |
| Gamma-glutamyl transpeptidase | |
| Anion gap | |
| Alpha₁Antitrypsin | |

Comparison were also made on the serum pH, protein and enzyme activities In human serum when treated with butanol-DIPE (40%-60% VN). The result are illustrated In the following table.

| | | **Control** | **Delipidated** |
|---|---|---|---|
| IgA | Mg/100ml | 168 | 167 |
| Igm | Mg/100ml | 144 | 144 |
| Ceruloplasmin | Mg/100ml | 1402 | 1395 |
| Transferrin | Mg/100ml | 30 | 31 |
| Albumin | g/100ml | 5.12 | 5.12 |
| Total protein | g/100ml | 7.35 | 7.42 |
| PH | | 7.37 | 7.37 |
| GOT | IU | 25 | 23 |
| AP | IU | 81 | 80 |
| a-amylase | IU | 293 | 293 |

It can be seen that treatment with the solvent system of the present invention does not adversely affect the above blood constituents. Importantly, there appears to be no denaturation of plasma proteins and change In enzyme activity, including the activity of lipid associated enzymes such as lecithin cholesterol acyltransferase and cholesterol ester transfer protein.

### Experiment 2

Cell free culture supernatant with serum containing approximately 100 infectious doses was mixed with butanol:diisopropyl ether (40:60) in a 1:2 ratio and mixed on an orbital shaker at 30 rpm for 1 hr.

The mixture was centrifuged at 400 x g for 10 min and the aqueous phase was removed. It was then mixed with diethyl ether and centrifuged as before, twice. Residual diethyl ether was removed by vacuum. A T-lymphocyte cell line was incubated with treated, untreated virus, or with no virus for 2 hours, then the cells were washed to remove virus and grown for two weeks. An ELISA assay to detect virus p24 antigen, showed that no virus replication took place in the cells infected with treated serum whereas virus replication took place in the cells treated with infected but untreated serum.

These results show that treatment of serum by the method and solvent system of the present invention can deactivate and eliminate infectivity of the HIV virus. Still further, this deactivation can be achieved without any adverse affects on the other serum components.

### Experiment 3

### Objective

To test whether the delipidation of serum results in inactivation of Duck Hepatitis B virus (DHBV).

A standard serum pool (Camden) containing 10⁶ID₅₀ doses of DHBV was used.

21 ducklings were obtained from a DHBV negative flock on day of hatch. These ducklings were tested at purchase and shown to be DHBV DNA negative by dot-blot hybridisation.

### Test Procedure

The organic solvent system was mixed in the ratio of 40% butanol to 60% diisopropyl ether. 4ml of the mixed organic solvent system was mixed with 2 ml of the standard serum pool and gently rotated for 1 hour. The mixture was centrifuged at 400xg for 10 minutes and the aqueous phase removed. It was then mixed with an equal volume of diethyl ether and centrifuged as before. The aqueous phase was then removed and mixed with an equal volume of diethyl ether and recentrifuged. The aqueous phase was removed and residual diethyl ether was removed by airing in a fume cabinet.

### Positive control and negative controls

The positive and negative contact sera were diluted In phosphate buffered saline (PBS).

Positive controls: 2ml of pooled serum containing 10⁶ID₅₀ doses of DHBV was mixed with 4 ml of PBS. Negative controls: 2ml of pooled DHBV negative serum was mixed with 4 ml of PBS.

Residual infectivity was tested by inoculation of 100µl of either test sample (n=7), negative (n=7) or positive (n=7) control into the peritoneal cavities of day-old ducks.

### Results

One of the positive control ducks died between 4 and 6 days of age and was excluded from further analysis. A further 3 positive control ducks died between 9 and 10 days of agent, and two treatment and one negative control died on day 11. It was decided to terminate the experiment. The remaining ducklings were euthanased on day 12 and their livers removed for DHBV DNA analysis as described by Deva et al (1995).

### Negative Controls

All seven negative control ducks remained DHBV negative.

### Positive Controls

All six positive control ducks were found to be DHBV positive in the liver.

### Test Ducks

All seven test ducks remained negative for DHBV DNA in their liver.

### Conclusion

Delipidation of serum using the above solvent system resulted in Inactivation of DHBV with none of the ducklings receiving treated serum becoming infected. Although the experiment had to be terminated on day 12 instead of day 14 all the positive control ducks were positive for DHBV (3/3 were DHBV positive by day 10). This suggests that sufficient time had elapsed for the treated ducks to become DHBV positive in the liver and that the premature ending of the experiment had no bearing on the results.

### Experiment 4

Inactivation of cattle pestivirus (bovine viral diarrhoea virus, BVDV), as a model for Hepatitis C.

### 1. Virus

A standard cattle pestivirus isolate (BVDV) was used in these experiments. This isolate, "Numerella" BVD virus, was isolated in 1987 from a diagnostic specimen submitted from a typical case of 'Mucosal Disease' on a farm in the Bega district of NSW. This virus is non-cytopathogenic, like 95% of BVDV isolates tested in our laboratory over a period of 30 years, and reacts with all 12 of a panel of monoclonal antibodies raised at EMAI as typing reagents. Therefore, this virus represents a 'standard strain' of Australian BVD viruses.

The Numerella virus was grown in bovine MDBK cells tested free of adventitious viral agents, including BVDV. The medium used for viral growth contained 10% Adult Bovine Serum derived from EMAI cattle, all tested free of BVDV virus and antibodies. This serum supplement has been employed in our laboratory for 30 years to exclude the possibility of adventitious BVDV contamination of test systems, a common failing in laboratories worldwide that do not take precautions to ensure the test virus is the only one in the culture system. Using these tested culture systems ensured high level replication of the virus and a high yield of infectious virus. Titration of the final viral yield after 5 days growth in MDBK cells showed a titre of 10^{6.8} infectious viral particles per ml of clarified (centrifuged) culture medium.

### 2. Inactivation of Infectious BVDV

100ml of tissue-culture supernatant, containing 10^{6.8} viral particles/ml, was harvested from a 150cm² tissue-culture flask. The supernatant was clarified by centrifugation (cell debris pelleted at 3000 rpm, 10min, 4°C) and 10ml set aside as a positive control for animal inoculation (non-inactivated virus). The remaining 90ml (containing 10^{7.75} infectious virus) was inactivated using the following protocol. Briefly, 180ml butanol:diisopropyl ether (2:1) was added and mixed by swirling. The mixture, in a 500ml conical flask, was then shaken for 30 min at 30rpm at room temperature on an orbital shaker. It was then centrifuged for 10min at 400xg and the organic solvent phase removed and discarded. In subsequent steps, the bottom layer (aqueous phase) may be removed from beneath the organic phase, improving yields considerably.

The aqueous phase, after butanol:diisopropyl ether treatment, was washed 4 times with an equal volume of fresh diethyl ether to remove all contaminating traces of butanol. Each time, the flask was swirled to ensure even mixing of the aqueous and solvent phases before centrifugation as above (400xg, 10min, 4°C). After 4 washes, the aqueous phase was placed In a sterile beaker in a fume hood overnight (16hr), covered with a sterile tissue to prevent contamination. The fume hood was left running to remove all remaining volatile ether residue from the inactivated viral preparation. It was then stored at 4°C under sterile conditions until inoculated into tissue culture or animals to test for any remaining infectious virus.

### 3. Testing of inactivated BVDV preparation

### 3.1 Tissue-culture inoculation

2ml of the solvent-inactivated virus preparation (10^{7.1} viral equivalents) was mixed with 8ml tissue-culture medium and adsorbed for 60min onto a monolayer of MDBK cells in a 25cm² tissue-culture flask. As a positive control, 2ml of non-inactivated virus (containing the same amount of live, infectious virus) was similarly adsorbed on MDBK cells in a 25cm²tissue-culture flask. After 60min, the supernatant was removed from both flasks and replaced with normal growth medium (+10% ABS). The flasks were then grown for 5 days under standard conditions before the MDBK cells were fixed and stained using a standard immunoperoxldase protocol with a mixture of 6 BVDV-specific monoclonal antibodies (EMAI panel, reactive with 2 different BVD viral proteins).

There were no infected cells in the monolayer of MDBK cells that was inoculated with the organic-solvent treated (inactivated) virus. In contrast, approximately 90% of the cells in the control flask (that was inoculated with non-inactivated BVD virus) were positive for virus as shown by heavy, specific, immunoperoxidase staining. These results showed that, under *in vitro* testing conditions, no infectious virus remained In the inactivated BVDV preparation.

### 3.2 Animal Innoculation

An even more sensitive *in vivo* test is to Inoculate naïve (antibody-negative) cattle with the inactivated-virus preparation. As little as one infectious viral particle injected subcutaneously In such animals is considered to be an infectious-cow dose, given that entry into cells and replication of the virus is extremely efficient for BVDV.

A group of 10 antibody-negative steers (10-12 months of age) were randomly allocated to 3 groups. The first group of 6 steers was used to test whether the BVD virus had been fully inactivated. Two steers were inoculated with non-inactivated virus to act as a positive-control while the 2 remaining steers acted as negative "sentinel" animals to ensure there was no natural pestivirus transmission occurring naturally within the innoculated group of animals. The positive control animals (inoculated with live, infectious virus) were run under separate, quarantined, conditions to stop them infecting any other animals when they developed a transient viraemia after infection (normally at 4-7 days after receiving live BVDV virus). Antibody levels were measured in all 10 animals using a validated, competitive ELISA developed at EMAI. This test has been Independently validated by CSL Ltd and is marketed by IDEXX Scandinavia in Europe.

The 6 animals in the first group each received a subcutaneous injection of 4.5ml of the inactivated BVDV preparation, incorporated in a commercial adjuvant. Since each ml of the inactivated preparation contained 10^{6.8} viral equivalents, the total viral load before inactivation was 10^{7.4} TCID₅₀. The positive-control animals received 5ml each of the non-inactivated preparation, that is, 10^{7.5} TCID₅₀ injected subcutaneously in the same way as for the first group. The remaining 2 'sentinel' animals were not given any viral antigens, being grazed with the first group of animals throughout the trial to ensure there was no natural pestivirus activity occurring in the group while the trial took place.

There was no antibody development in any of the steers receiving the inactivated BVD virus preparation. At 2 and 4 weeks after a single dose, none of the 6 steers seroconverted showing that there was no infectious virus left in a total volume of 27ml of the inactivated virus preparation. This is the equivalent of a total inactivation of 10^{8.2} TCID₅₀. In contrast, there were high levels of both anti-E2 antibodies (neutralizing antibodies) and anti-NS3 antibodies at both 2 and 4 weeks after inoculation in the 2 animals receiving 5ml each of the viral preparation prior to inactivation. This confirmed the infectious nature of the virus prior to inactivation. These In vivo results confirm the findings of the *in vitro* tissue-culture test. The 2 'sentinel' animals remained seronegative throughout showing the herd remained free of natural pestivirus Infections.

### Experiment 5

An Election Microscope study was conducted with the object of viewing virus particles before and after inactivation.

Inactivation was conducted with DIPE/Butanol and DIPE alone for 60 min, 1 min and 30 seconds. There was no infectivity or visible virus particles detected by EM, even after treatment for 30 seconds. No virus particles at all were observed for the inactivated samples. It is believed that destruction of the virus liquid envelope occurs too rapidly for observation.

It can be seen that the methods and solvent system of the present invention can rapidly and efficiently inactivate infectious agents including the HIV virion in biological fluids.

Further, such inactivation occurs without appreciable destruction of proteins which can have adverse effects on human health. Still further, dissolved lipids are removed and are not introduced into a patient.

The present invention can also rapidly and efficiently inactivate infectious agents in biological fluids and blood products. The method is relatively simple and does not require complex procedures and equipment for removal of the solvent system as compared with for example the currently used SD plasma treatment method as previously described. A relatively simple method of inactivating a virus is desirable on an economic level and also has wider potential in developing countries and particularly those where HIV is prevalent.

In the present specification and claims it will be understood that the terms "comprises" and "comprising" are not limited to the stated integer or integers and does not exclude one or more other integers.

## Claims

1. A method of reducing the activity of an infectious agent having a lipid envelope or membrane, the method comprising:
contacting a biological fluid with a solvent system comprising a solvent in which lipids are soluble and in which.hematological and biochemical constituents are substantially stable and which is immiscible with the biological fluid, for a time sufficient to reduce active levels of the infectious agent in the fluid; and
separating the fluid from the solvent system, whereby dissolved lipids are separated with and remain in the solvent system,
**characterized in that**
the solvent system comprises at least one alcohol, ether, hydrocarbon, amine, ester, or mixture thereof,

2. A method according to claim 1, whereby the biological fluid is blood, blood plasma, blood plasma fractions, blood cell derivatives such as hemoglobin, alpha-interferon, growth factors, T-cell growth factor, platelet derived growth factor, plasminogen activator, blood plasma precipitates including cryoprecipitate, ethanol precipitate, and polyethylene glycol precipitate, or supernatants such as cryosupernatant, ethanol supernatant and polyethylene glycol supernatant, mammalian semen and serum, particularly fetal calf serum.

3. A method according to claims 1 or 2, whereby the solvent system is substantially immiscible in the fluid or blood product and separation of the solvents system from the fluid or blood product includes allowing the solvent system and fluid or blood product to settle so as to form a solvent layer containing lipids and an aqueous plasma layer, and separating the two layers.

4. The method of any of claims 1 to 3, wherein the infectious agent is a virus.

5. The method of any of claims 1 to 4, wherein the infectious agent is selected from the HIV, Hepatitis B or Hepatitis C virus. Alphavirus (alphaviruses), Rubivirus (rubella virus), Flavivirus (flaviviruses), Pestivirus (mucosal disease viruses), unnamed Hepatitis C virus, Coronavirus (coronaviruses), Torovirus (toroviruses), Arterivirus (arteriviruses), Paramyxovirus (paramyxoviruses), Rubulavirus (ribulaviruses). Morbillivirus (morbiliviruses), Pneumovirinae (pneumoviruses), Pneumovirus (pneumoviruses), Vesiculovirus (vesiculoviruses), Lyssavirus (lyssaviruses), Ephemerovirus (ephemeroviruses), Cytorhabdovirus (plant rhabdovirus group A), Nucleorhabdovirus (plant rhabdovirus group B), Filovirus (filoviruses), Influenzavirus A, B (influenza A and B viruses), Influenzavirus C (influenza C virus), unnamed Thogoto-like viruses, Bunyavirus (bunyaviruses). Phlebovirus (phleboviruses), Nairovirus (nairoviruses), Hantavirus (hantaviruses), Tospovirus (tospoviruses), Arenavirus (arenaviruses), unnamed mammalian type B retroviruses, unnamed mammalian and reptilian type C retroviruses, unnamed type D retroviruses, Lentivirus (lentiviruses), Spumavirus (spumaviruses), Orthohepadnavirus (hepadnaviruses of mammals). Avihepadnavirus (hepadnaviruses of birds), Simplexvirus (simplexviruses), Varicellovirus (varicelloviruses),

6. The method of any of claims 1 to 5
wherein the alcohol is a C₁-C₈ alcohol.

7. The method of claim 6, wherein the alcohol is butanol.

8. The method of any of claims 1 to 7, wherein the ether is selected from the group consisting of ethyl propyl ether, propyl ether, diisopropyl ether, diethyl ether or a mixture thereof.

9. The method of claim 8, wherein the ether is diisopropyl or diethyl ether.

10. The method of any of claims 7 to 9, wherein the solvent system comprises about 0% to about 60% alcohol and between about 40 to about 100% ether.

11. The method of any of claims 1 to 10, wherein the aqueous plasma layer is washed with an ether to remove residual solvent and/or to de-emulsify the aqueous layer.

## Patentansprüche

1. Verfahren zum Verringern der Aktivität eines infektiösen Mittels mit einer Lipidhülle oder -membran, wobei das Verfahren umfasst:
In-Kontakt-bringen eines biologischen Fluids mit einem Lösungsmittelsystem, umfassend ein Lösungsmittel, worin Lipide löslich sind und worin hämatologische und biochemische Bestandteile im Wesentlichen stabil sind und das nicht mischbar ist mit einem biologischen Fluid, für eine Zeit, die ausreichend ist, um die aktiven Gehalte des infektiösen Mittels in dem Fluid zu verringern; und
Trennen des Fluids von dem Lösungsmittelsystem, wobei gelöste Lipide mit dem Lösungsmittelsystem abgetrennt werden und darin verbleiben,
**dadurch gekennzeichnet, dass**
das Lösungsmittelsystem mindestens einen Alkohol, Ether, Kohlenwasserstoff, ein Amin, einen Ester oder ein Gemisch davon umfasst.

2. Verfahren nach Anspruch 1, wobei das biologische Fluid Blut, Blutplasma, Blutplasmafraktionen, Blutzellderivate, wie etwa Hämoglobin, alpha-Interferon, Wachstumsfaktoren, T-Zell-Wachstumsfaktor, Plättchen-abgeleiteter Wachstumsfaktor, Plasminogenaktivator, Blutplasmapräzipitate, einschließlich Kryopräzipitat, Ethanolpräzipitat und Polyethylenglykolpräzipitat, oder Überstände, wie etwa Kryoüberstand, Ethanolüberstand und Polyethylenglykolüberstand, Säugersamen und -serum, insbesondere fötales Kalbserum, ist.

3. Verfahren nach Anspruch 1 oder 2, wobei das Lösungsmittel im Wesentlichen nicht mischbar ist in dem Fluid oder Blutprodukt und eine Trennung des Lösungsmittelsystems von dem Fluid oder Blutprodukt umfasst, dass zugelassen wird, dass das Lösungsmittelsystem und das Fluid- oder Blutprodukt sich absetzen, um eine Lösungsmittelschicht zu bilden, die Lipide enthält, und eine wässrige Plasmaschicht zu bilden, und Trennen der beiden Schichten.

4. Verfahren nach einem der Ansprüche 1 bis 3, worin das infektiöse Mittel ein Virus ist.

5. Verfahren nach einem der Ansprüche 1 bis 4, worin das infektiöse Mittel ausgewählt wird aus dem HIV-, Hepatitis B- oder Hepatitis C-Virus, Alphavirus (Alphaviren), Rubivirus (rubella virus), Flavivirus (Flaviviren), Pestvirus (Schleimhautkrankheit-Viren), Unbenannter Hepatitis C-Virus, Coronavirus (Coronaviren), Torovirus (Toroviren), Arterivirus (Arteriviren), Paramyxovirus (Paramyxoviren), Rubulavirus (Ribulaviren), Morbillivirus (Morbilliviren), Pneumovirlrinae (Pneumoviren), Pneumovirus (Pneumoviren), Vesiculovirus (Vesiculoviren), Lyssavirus (Lyssaviren), Ephemerovirus (Ephemoroviren), Cytorhabdovirus (Pflanzen-Rhabdovirus Gruppe A), Nucleorhabdovirus (Pflanzen-Rhabdovirus Gruppe B), Filovirus (Filoviren), Influenzavirus A, B (Influenza A- und B-Viren), Influenzavirus C (Influenza C-Virus), Unbenannte Thogoto-ähnliche Viren, Bunyavirus (Bunyaviren), Phlebovirus (Phleboviren), Nairovirus (Nairoviren), Hantavirus (Hantaviren), Tospovirus (Tospoviren), Arenavirus (Arenaviren), Unbenannte Säuger-Typ B-Retroviren, unbenannte Säuger- und Reptilien Typ-C-Retroviren, Unbenante Typ D-Retroviren, Lentivirus (Lentiviruses), Spumavirus (Spumaviren), Orthohepadnavirus (Hepadna-Viren von Säugern), Avihepadnavirus (Hepadnaviren von Vögeln), Simplexvirus (Simplexviren), Varicellovirus (Varicelloviren).

6. Verfahren nach einem der Ansprüche 1 bis 5, worin der Alkohol ein C₄-C₈-Alkohol ist.

7. Verfahren nach Anspruch 6, worin der Alkohol Butanol ist.

8. Verfahren nach einem der Ansprüche 1 bis 7, worin der Ether ausgewählt wird aus der Gruppe, bestehend aus Ethylpropylether, Propylether, Diisopropylether, Diethylether oder einem Gemisch davon.

9. Verfahren nach Anspruch 8, worin der Ether Diisopropylether- oder Diethylether ist.

10. Verfahren nach einem der Ansprüche 7 bis 9, worin das Lösungsmittelsystem etwa 0 % bis etwa 60 % Alkohol und zwischen etwa 40 bis etwa 100 % Ether umfasst.

11. Verfahren nach einem der Ansprüche 1 bis 10, worin die wässrige Plasmaschicht mit einem Ether gewaschen wird, um restliches Lösungsmittel zu entfernen und/oder die wässrige Schicht zu entemulgieren.

## Revendications

1. Procédé pour réduire l'activité d'un agent infectieux ayant une enveloppe ou une membrane lipidique, ce procédé renfermant les étapes consistant à :
- mettre en contact un fluide biologique avec un système de solvant renfermant un solvant dans lequel les lipides sont solubles, dans lequel les constituants hématologiques et biochimiques sont essentiellement stables, et qui est non miscible avec le fluide biologique pendant un laps de temps suffisant pour réduire les niveaux d'activité de l'agent infectieux dans le fluide, et
- séparer le fluide du système de solvant, les lipides dissouts étant séparés du système de solvant et restant dans ce système,
**caractérisé en ce que**
le système de solvant renferme au moins un alcool, un éther, un hydrocarbure, une amine, un ester, ou un mélange de ces composés.

2. Procédé conforme à la revendication 1,
selon lequel
le fluide biologique est le sang, le plasma sanguin, des fractions de plasma sanguin, des dérivés de cellules sanguines tels que l'hémoglobine, l'alpha interféron, les facteurs de croissance, le facteur de croissance des cellules T, le facteur de croissance dérivé des plaquettes sanguines, l'activateur du plasminogène, les précipités du plasma sanguin incluant les cryoprécipités, les précipités à l'éthanol et les précipités au polyéthylène glycol, ou des surnageants tels que les cryosurnageants, les surnageants à l'éthanol, et les surnageants au polyéthylène glycol, le sperme et le sérum de mammifères, en particulier le sérum de veau foetal.

3. Procédé conforme à la revendication 1 ou à la revendication 2,
selon lequel
le système de solvant est essentiellement non miscible dans le fluide ou le produit sanguin, et la séparation du système de solvant du fluide ou du produit sanguin renferme une étape selon laquelle on laisse au repos le système de solvant et le fluide ou le produit sanguin de façon à former une couche de solvant renfermant des lipides et une couche de plasma aqueux et on sépare ces deux couches.

4. Procédé conforme à l'une quelconque des revendications 1 à 3,
selon lequel
l'agent infectieux est un virus.

5. Procédé conforme à l'une quelconque des revendications 1 à 4,
selon lequel
l'agent infectieux est choisi dans le groupe formé par les virus suivants :
HIV, Hepatitis B or Hepatitis C virus, Alphavirus (alphavirus), Rubivirus (rubella virus), Flavivirus (flavivirus), Pestivirus (virus des affections des muqueuses), Hepatitis C virus non précisé, Coronavirus (coronavirus), Torovirus (torovirus), Arterivirus (arterivirus), Paramyxovirus (paramyxovirus), Rubulavirus (ribulavirus), Morbillivirus (morbillivirus), Pneumovirinae (pneumovirus), Pneumovirus (pneumovirus), Vesiculovirus (vesiculovirus), Lyssavirus (lyssavirus), Ephemerovirus (ephemerovirus), Cytorhabdovirus (rhabdovirus des plantes du groupe A), Nucleorhabdovirus (rhabdovirus des plantes du groupe B), Filovirus (filovirus), Influenzavirus A, B (influenza A and B virus), Influenza virus C (influenza C virus) virus de type Thogoto non précisés, Bunyavirus (bunyavirus), Phlebovirus (Phlebovirus), Nairovirus (nairovirus), Hantavirus (hantavirus), Tospovirus (tospovirus), Arenavirus (arenavirus), rétrovirus de mammifères du type B non précisés, rétrovirus de mammifères et de reptiles du type C non précisé du type D non précisés rétroviruses, Lentivirus (lentivirus), Spumavirus (spumavirus), Orthohepadnavirus (hepadnavirus de mammifères), Avihepadnavirus (hepadnavirus d'oiseaux), Simplexvirus (simplexvirus), Varicellovirus (varicellovirus).

6. Procédé conforme à l'une quelconque des revendications 1 à 5,
selon lequel
l'alcool est un alcool en C₁ - C₃.

7. Procédé conforme à la revendication 6,
selon lequel
l'alcool est le butanol.

8. Procédé conforme à l'une quelconque des revendications 1 à 7,
selon lequel
l'éther est choisi dans le groupe formé par les éthers suivants : éthyl propyl éther, propyl éther, diisopropyl éther, diéthyl éther ou leurs mélanges.

9. Procédé conforme à la revendication 8,
selon lequel
l'éther est le diisopropyl ou le diéthyl éther.

10. Procédé conforme à l'une quelconque des revendications 7 à 9,
selon lequel
le système de solvant renferme entre environ 0 % et environ 60 % d'alcool et entre environ 40 % et environ 100 % d'éther.

11. Procédé conforme à l'une quelconque des revendications 1 à 10,
selon lequel
la couche de plasma aqueux es lavée avec un éther pour éliminer le solvant résiduel et/ou pour dé-émulsifier la couche aqueuse.
